(19) Europäisches Patentamt
European Patent Office
Office européen des brevets

(11) EP 3 668 316 B1

(12) # EUROPEAN PATENT SPECIFICATION

(45) Date of publication and mention of the grant of the patent:
**15.11.2023 Bulletin 2023/46**

(21) Application number: **18732805.9**

(22) Date of filing: **27.06.2018**

(51) International Patent Classification (IPC):
***A01N 33/04*** *(2006.01)* ***A01N 35/02*** *(2006.01)*
***A61K 8/35*** *(2006.01)* ***A61K 8/41*** *(2006.01)*
***A01P 1/00*** *(2006.01)*

(52) Cooperative Patent Classification (CPC):
**A01N 33/04; A01N 35/02; A61K 8/35; A61K 8/41**

(86) International application number:
**PCT/EP2018/067300**

(87) International publication number:
**WO 2019/034316 (21.02.2019 Gazette 2019/08)**

(54) **ANTIMICROBIAL MIXTURE CONTAINING 4-(3-ETHOXY-4-HYDROXYPHENYL)BUTAN-2-ONE AND AN AMMONIUM COMPOUND, AND COSMETIC COMPOSITION CONTAINING SAME**

ANTIMIKROBIELLE MISCHUNG MIT 4-(3-ETHOXY-4-HYDROXYPHENYL)BUTAN-2-ON UND EINER AMMONIUMVERBINDUNG UND KOSMETISCHE ZUSAMMENSETZUNG DAMIT

MÉLANGE ANTIMICROBIEN CONTENANT DE LA 4-(3-ÉTHOXY-4-HYDROXYPHÉNYL)BUTAN-2-ONE ET UN COMPOSÉ D’AMMONIUM, ET COMPOSITION COSMÉTIQUE LE CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.08.2017 FR 1757744**
**18.08.2017 FR 1757746**

(43) Date of publication of application:
**24.06.2020 Bulletin 2020/26**

(73) Proprietor: **L’OREAL**
**75008 Paris (FR)**

(72) Inventors:
- **MALET, Gael**
**94152 Chevilly Larue (FR)**
- **CUPFERMAN, Sylvie**
**94152 Chevilly Larue (FR)**
- **MENARD-SZCZEBARA, Florence**
**94152 Chevilly Larue (FR)**

(74) Representative: **L’Oreal**
**Service D.I.P.I.**
**9, rue Pierre Dreyfus**
**92110 Clichy (FR)**

(56) References cited:
**WO-A1-2011/039445 US-A1- 2005 019 431**
**US-A1- 2015 265 666**

- **P GILBERT ET AL: "Antimicrobial activity of some alkyltrimethylammonium bromides", LETTERS IN APPLIED MICROBIOLOGY, vol. 1, 1 January 1985 (1985-01-01), pages 101-104, XP055433660, DOI: 10.1111/j.1472-765X.1985.tb01498.x**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 668 316 B1

## Description

A subject of the present invention is an antibacterial mixture containing 4-(3-ethoxy-4-hydroxyphenyl)butan-2-one and a particular ammonium compound, and also after a cosmetic composition containing such a mixture.

4-(3-Ethoxy-4-hydroxyphenyl)butan-2-one (ketone compound) is a useful substance as a preserving agent for cosmetic compositions, for protecting the compositions against microbial contamination, as described in patent application WO 2011/039445.

Ammonium compounds are known in the field of cosmetic and microbiology, see for eg. the documents US 2005/019431 A1, US 2015/265666 A1 and « Antimicrobial activity of some alkyltrimethylammonium bromides" Letters in Applied Micriobiology, P Gilbert et al : vol. 1, , p. 101-104, (1985).

However, it is desirable to be able to incorporate said ketone compound in reduced concentration in compositions, especially cosmetic or dermatological compositions, while at the same time maintaining good antimicrobial conservation performance. Combinations of the ketone compound with other preserving agents that have good antimicrobial efficacy are thus sought for this purpose.

The inventors have discovered, unexpectedly, that the combination of 4-(3-ethoxy-4-hydroxyphenyl)butan-2-one with an ammonium compound chosen from a fatty-chain alkyltrimethylammonium salt, in particular behenyltrimethylammonium chloride, makes it possible to obtain an antimicrobial mixture with synergistic antimicrobial activity, in particular at least on moulds, especially on *Aspergillus niger.*

The results of the examples described below show the synergistic antimicrobial activity obtained with the minimum inhibitory concentration (MIC) measurements taken with several mixtures. The antimicrobial activity is considered as being synergistic when the antimicrobial mixture makes it possible to obtain a percentage of strain growth of less than or equal to 25%, or even less than or equal to 20%.

The combination of 4-(3-ethoxy-4-hydroxyphenyl)butan-2-one with a particular fatty-chain alkyltrimethylammonium salt as defined below, in particular behenyltrimethylammonium chloride, makes it possible to obtain an antimicrobial mixture with synergistic antimicrobial activity, in particular on moulds, especially on *Aspergillus niger*, on yeasts, in particular on *Candida albicans*, and on gram-negative bacteria, especially on *Pseudomonas aeruginosa.*

The combination of 4-(3-ethoxy-4-hydroxyphenyl)butan-2-one with an alkyldimethylammonium salt as defined below, makes it possible to obtain an antimicrobial mixture with synergistic antimicrobial activity, in particular on moulds, especially on *Aspergillus niger.*

More precisely, a subject of the invention is an antimicrobial mixture comprising, or constituted by (or consisting of), 4-(3-ethoxy-4-hydroxyphenyl)butan-2-one and an ammonium compound chosen from:
i) an alkyltrimethylammonium salt of formula $R\text{-}N^+(CH_3)_3\ X^-$ (I) in which R denotes an alkyl group containing from 20 to 24 carbon atoms, $X^-$ denotes a halide ion, in particular chloride or bromide, or a methosulfate ion;

A subject of the invention is also a composition, especially a cosmetic composition, comprising, in a physiologically acceptable medium, said mixture described previously.

A further subject of the invention is a process for the non-therapeutic cosmetic treatment of keratin materials, comprising the application to the keratin materials of a composition as described previously. The process may be a cosmetic process for caring for or making up or cleansing keratin materials.

A subject of the invention is also a process for conserving a composition comprising a physiologically acceptable medium, in particular a cosmetic or dermatological composition, characterized in that it consists in incorporating into said composition an antimicrobial mixture as described previously.

A subject of the invention is also the use of the antimicrobial mixture described previously for conserving a composition comprising a physiologically acceptable medium.

4-(3-Ethoxy-4-hydroxyphenyl)butan-2-one is a compound of formula:

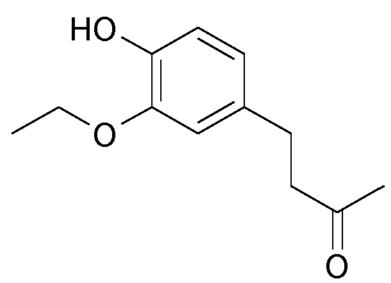

A subject of the invention is an antimicrobial mixture comprising, or constituted by (or consisting of), 4-(3-ethoxy-4-hydroxyphenyl)butan-2-one and an alkyltrimethylammonium salt of formula $R\text{-}N^+(CH_3)_3\ X^-$ (I) in which R denotes an alkyl group containing from 20 to 24 carbon atoms, $X^-$ denotes a halide ion, in particular chloride or bromide, or a

methosulfate ion.

For the alkyltrimethylammonium salt of formula (I) described previously, R is preferably a behenyl group.

Preferably, $X^-$ is a chloride anion.

Advantageously, the alkyltrimethylammonium salt (I) is behenyltrimethylammonium chloride (INCI name: behentrimonium chloride).

Behenyltrimethylammonium chloride may be used as a mixture at 79% by weight with 18% of isopropyl alcohol and 3% of water sold under the name Genamin KDMF by the company Clariant.

Advantageously, 4-(3-ethoxy-4-hydroxyphenyl)butan-2-one and the trimethylammonium salt (I) are present in said mixture in a content such that the 4-(3-ethoxy-4-hydroxyphenyl)butan-2-one/salt (I) (preferably behenyltrimethylammonium chloride) weight ratio ranges from 0.08 to 80, preferably from 0.45 to 70 and preferentially from 1.5 to 50.

The antimicrobial mixture according to the invention has synergistic antimicrobial activity, in particular on moulds, especially on *Aspergillus niger*, on yeasts, in particular on *Candida albicans*, and on gram-negative bacteria, in particular on *Pseudomonas aeruginosa.*

Preferentially, the antimicrobial mixture according to the invention contains behenyltrimethylammonium chloride.

The antimicrobial mixture may have a 4-(3-ethoxy-4-hydroxyphenyl)butan-2-one/salt (I) (preferably behenyltrimethylammonium chloride) weight ratio ranging from 10 to 60, preferably ranging from 10 to 55 and preferentially ranging from 15 to 40, and better still ranging from 20 to 30. Such a mixture has good antimicrobial activity on yeasts, especially on *Candida albicans.*

The antimicrobial mixture may have a 4-(3-ethoxy-4-hydroxyphenyl)butan-2-one/salt (I) (preferably behenyltrimethylammonium chloride) weight ratio ranging from 0.08 to 5, preferably ranging from 0.45 to 4.5, preferentially ranging from 0.45 to 3, better still from 0.4 to 2.5, and preferentially ranging from 1.5 to 3. Such a mixture has good antimicrobial activity on moulds, especially on *Aspergillus niger.*

The antimicrobial mixture may have a 4-(3-ethoxy-4-hydroxyphenyl)butan-2-one/salt (I) (preferably behenyltrimethylammonium chloride) weight ratio ranging from 3 to 80, preferably ranging from 3 to 70, preferentially ranging from 3 to 50 and better still ranging from 3 to 45. Such a mixture has good antimicrobial activity on gram-negative bacteria, especially on *Pseudomonas aeruginosa.*

A subject of the invention is also a composition comprising, in a physiologically acceptable medium, the antimicrobial mixture described previously.

The term "physiologically acceptable medium" means a medium that is compatible with human keratin materials such as the skin, the scalp, the hair and the nails. Said medium may comprise one or more additional ingredients other than the ketone compound and the ammonium compound described previously.

The compound 4-(3-ethoxy-4-hydroxyphenyl)butan-2-one may be present in the composition according to the invention at an amount ranging from 0.01% to 5% by weight relative to the total weight of the composition, preferably ranging from 0.01 % to 3% by weight, preferentially ranging from 0.01% to 2.5% by weight and more preferentially ranging from 0.01% to 2% by weight.

The composition may comprise at least one additional ingredient chosen from water, oils, polyols containing from 2 to 10 carbon atoms, gelling agents, surfactants, film-forming polymers, dyestuffs, fragrances, fillers, UV-screening agents, plant extracts, cosmetic and dermatological active agents, and salts.

The composition according to the invention may comprise an aqueous phase.

The composition may comprise water, which may be present at a content ranging from 5% to 90% by weight relative to the total weight of the composition, and preferably ranging from 35% to 75% by weight.

The composition may also comprise a polyol that is water-miscible at room temperature (25°C), especially chosen from polyols especially containing from 2 to 10 carbon atoms, preferably containing from 2 to 6 carbon atoms, such as glycerol, propylene glycol, 1,3-propanediol, butylene glycol, pentylene glycol, hexylene glycol, dipropylene glycol, diethylene glycol or diglycerol. Advantageously, the composition according to the invention comprises 1,3-propanediol, especially in a content ranging from

0.1% to 20% by weight, preferably ranging from 0.1% to 10% by weight and preferentially ranging from 0.5% to 5% by weight, relative to the total weight of the composition.

The compositions according to the invention may be in the form of oil-in-water (O/W) emulsions, water-in-oil (W/O) emulsions or multiple emulsions (triple: W/O/W or O/W/O), oily solutions, oily gels, aqueous solutions, aqueous gels, solid compositions. These compositions are prepared according to the usual methods.

The compositions according to the invention may be more or less fluid and may have the appearance of a white or coloured cream, an ointment, a milk, a lotion, a serum, a paste or a foam. They may be optionally applied to the skin in aerosol form. They may also be in solid form, for example in the form of a stick or a compact powder.

The composition according to the invention may especially be in the form of:

- a makeup product, especially for making up the skin of the face, the body, or the lips or the eyelashes;

- an aftershave gel or lotion; a shaving product;
- a deodorant (stick, roll-on or aerosol);
- a hair-removing cream;
- a body hygiene composition such as a shower gel or a shampoo;
- a pharmaceutical composition;
- a solid composition such as a soap or a cleansing bar;
- an aerosol composition also comprising a pressurized propellant;
- a hairsetting lotion, a hair-styling cream or gel, a dye composition, a permanent-waving composition, a lotion or a gel for combating hair loss, or a hair conditioner;
- a composition for caring for or cleansing the skin.

A subject of the invention is also a process for preparing a composition, especially a cosmetic or dermatological composition, comprising a step of mixing 4-(3-ethoxy-4-hydroxyphenyl)butan-2-one, the ammonium compound described previously, and one or more additional ingredients, especially cosmetic or dermatological ingredients, such as those described previously.

The invention is illustrated in greater detail in the example that follows. The amounts of the ingredients are expressed as weight percentages.

**Example 1: determination of the synergistic antimicrobial activity as MIC**

The demonstration of a synergistic antimicrobial activity effect with a mixture of 4-(3-ethoxy-4-hydroxyphenyl)butan-2-one (referred to as substance A) and of ammonium compound (referred to as substance B) is performed by calculating the synergy index (or FIC index).

**Formulation**

$$\text{FIC Index} = (\text{MICa with B}/\text{MICa}) + (\text{MICb with A}/\text{MICb})$$

with:

- MICa with B: minimum concentration of product A in the combination A + B which makes it possible to obtain an inhibitory effect

- MICb with A: minimum concentration of product B in the combination A + B which makes it possible to obtain the inhibitory effect.

- MICa: minimum inhibitory concentration of product A alone.

- MICb: minimum inhibitory concentration of product B alone.

This formula was described for the first time in the article by F.C. Kull, P.C. Eisman, H.D. Sylwestrowka, and R.L. Mayer, Applied Microbiology 9:538-541, 1961.

For each compound tested alone, the MIC is considered as the first concentration which makes it possible to obtain a microbial growth percentage of less than or equal to 25%.

As regards the combinations tested, MICa with b and MICb with a are the respective concentrations of A and of B in the combinations which make it possible to obtain a microbial growth percentage of less than or equal to 25%.

**Interpretation of the FIC Index:**

When the FIC index value is less than or equal to 1, it is considered that the combination of test compounds has a synergistic effect.

The results obtained are summarized in the following tables.

The combination of compounds A and B was tested on the following strains or a part of these strains: *Aspergillus niger*, *Candida albicans*, *Pseudomonas aeruginosa.*

The microbial strain *Aspergillus niger* ATCC 6275, and a double-concentration Sabouraud broth liquid culture medium supplemented with polyoxyethylenated (20 OE) sorbitan monopalmitate (Tween 40 from Croda) and Phytagel© BioReagent were used.

The microbial strain *Candida albicans* ATCC 10231 and a double-concentration Sabouraud broth liquid culture

medium were used.

The microbial strain *Pseudomonas aeruginosa* ATCC 9027 and a double-concentration nutritive broth liquid culture medium were used.

A 96-well microplate and an incubation time of 32.5°C are used.

The incubation time of the microplate is:

- from 24 hours to 48 hours aerobically for *Aspergillus niger,*
- from 18 to 24 hours aerobically for *Candida albicans* and *Pseudomonas aeruginosa.*

## Tests

For each compound:

A = 4-(3-ethoxy-4-hydroxyphenyl)-2-butanone compound
B = behenyltrimethylammonium chloride (as a mixture at 79% by weight with 18% of isopropyl alcohol and 3% of water sold under the name Genamin KDMF by the company Clariant*)
* It is considered that the isopropyl alcohol present has no influence on the MIC results obtained.

A 10% (weight/volume) stock solution was prepared by mixing 1 g of compound in 9 ml of aqueous 1‰ agar solution. Successive dilutions were made with the 1‰ agar solution.

- Tests of compounds A and B alone

50 μL of each of the daughter solutions obtained containing compound A or B are added to the microplate wells. 100 μL of Sabouraud liquid nutrient broth seeded with the strain *Aspergillus niger* and 50 μL of aqueous 1‰ agar solution are also added thereto.

- Tests of compounds A and B as a mixture

50 μL of each of the daughter solutions obtained containing compound A and 50 μL of each of the daughter solutions obtained containing compound B are added to the microplate wells. 100 μL of Sabouraud liquid nutrient broth seeded at double concentration with the strain *Aspergillus niger* are also added thereto.

## Microbial growth control

A positive microbial growth control was also prepared. The positive microbial growth control corresponds to a mixture of 100 μL of aqueous 1‰ agar solution with 100 μL of Sabouraud liquid nutrient broth seeded at double concentration with the strain *Aspergillus niger* in the absence of compounds A and B.

## Absorbance control of compounds A and B alone

An absorbance control was performed in parallel on compounds A and B alone. This control corresponds to 100 μL of double concentration sterile Sabouraud liquid nutrient broth + 100 μL of double concentration compound A or B.

In the three cases (absorbance control, growth control and test), the final volume present in each of the microplate wells is 200 μL.

In the two cases (test and control), the inoculum represents the concentration of the strain *Aspergillus niger* present in the final volume of the wells (200μL) and is between 2 and $6 \times 10^5$ cfu/ml of *Aspergillus niger.*

The minimum inhibitory concentration (MIC) of each compound A and B alone and in combination was determined in a known manner by means of optical density measurements at a wavelength of 620 nm.

The test as described above (tests, absorbance control and growth control) was performed again to test the combination A + B on the strains *Candida albicans* and *Pseudomonas aeruginosa.*

The following results were obtained with

B1 = behenyltrimethylammonium chloride (as a mixture at 79% by weight with 18% of isopropyl alcohol and 3% of water sold under the name Genamin KDMF by the company Clariant*)
* It is considered that the isopropyl alcohol present has no influence on the MIC results obtained.

*Candida albicans*

| Concentrations tested (in weight%) | 0 A | 0.025 A | 0.05 A | 0.1 A | **0.2 A** |
|---|---|---|---|---|---|
| 0 B1 | | 73 | 73 | 49 | 9 |
| 0.002 B1 | 50 | **13 (FIC 0.63)** | **8 (FIC 0.75)** | **14 (FIC 1)** | 1 |
| **0.00395 B1** | 1 | 0 | 0 | 1 | 1 |

| % MIC of A alone | % MIC of B1 alone | MIC of each compound as a mixture | | FIC Index | Ratio A/B1 |
|---|---|---|---|---|---|
| | | A % | B1 % | | |
| 0.2 | 0.00395 | 0.025 | 0.002 | 0.63 | 12.5 |

The results obtained show synergistic inhibitory activity for the mixtures:

i) 0.025% of A and 0.002% of B1, i.e. ratio A/B1 = 12.5
ii) 0.05% of A and 0.002% of B1, i.e. ratio A/B1 = 25
iii) 0.1% of A and 0.002% of B1, i.e. ratio A/B1 = 50

*Aspergillus niger*

| Concentrations tested (in weight%) | 0 A | 0.025 A | 0.05 A | 0.1 A | 0.2 A | **0.4 A** |
|---|---|---|---|---|---|---|
| 0 B1 | | 150 | 113 | 112 | 75 | 5 |
| 0.049 B1 | 186 | 138 | 178 | **-8 (FIC 0.31)** | **-7 (FIC 0.56)** | -15 |
| 0.1 B1 | 155 | 139 | 114 | **18 (FIC 0.37)** | **-7 (FIC 0.62)** | 8 |
| 0.2 B1 | 78 | 131 | 100 | **15 (FIC 0.49)** | **-21 (FIC 0.74)** | 9 |
| 0.4 B1 | 34 | 66 | **-14 (FIC 0.60)** | **19 (FIC 0.73)** | **-14 (FIC 0.98)** | -10 |
| **0.79 B1** | 8 | 20 | 26 | -35 | -20 | -50 |

| % MIC of A alone | % MIC of B1 alone | MIC of each compound as a mixture | | FIC Index | Ratio A/B1 |
|---|---|---|---|---|---|
| | | A % | B1 % | | |
| 0.4 | 0.79 | 0.1 | 0.049 | 0.31 | 2 |

The results obtained show synergistic inhibitory activity for the mixtures:

i) 0.05% of A and 0.4% of B1, i.e. ratio A/B1 = 0.125
ii) 0.1% of A and 0.4% of B1, i.e. ratio A/B1 = 0.25
iii) 0.1% of A and 0.2% of B1, i.e. ratio A/B1 = 0.5
iv) 0.1% of A and 0.1% of B1, i.e. ratio A/B1 = 1
v) 0.1 % of A and 0.049% of B1, i.e. ratio A/B1 = 2
vi) 0.2% of A and 0.4% of B1, i.e. ratio A/B1 = 0.5
vii) 0.2% of A and 0.2% of B1, i.e. ratio A/B1 = 1
viii) 0.2% of A and 0.1% of B1, i.e. ratio A/B1 = 2
ix) 0.2% of A and 0.049% of B1, i.e. ratio A/B1 = 4

*Pseudomonas aeruginosa*

| concentrations tested (in weight %) | 0 A | 0.0625 A | 0.125 A | 0.25 A | 0.5 A | **1 A** |
|---|---|---|---|---|---|---|
| 0 B1 | | 114 | 59 | 65 | 41 | 15 |
| 0.001975 B1 | 68 | 85 | 64 | 49 | 45 | 49 |
| 0.00395 B1 | 93 | 65 | 75 | 52 | 51 | 21 |
| 0.0079 B1 | 36 | 35 | 31 | 25 | **18 (FIC 0.75)** | 8 |
| 0.0158 B1 | 30 | **5 (FIC 0.56)** | **0 (FIC 0.63)** | **-1 (FIC 0.75)** | **0 (FIC 1)** | 40 |
| **0.0316 B1** | 1 | -3 | -2 | 0 | 4 | -34 |

| % MIC of A alone | % MIC of B1 alone | MIC of each compound as a mixture | | FIC Index | Ratio A/B1 |
|---|---|---|---|---|---|
| | | A % | B1 % | | |
| 1 | 0.0316 | 0.0625 | 0.0158 | 0.56 | 3.95 |

The results obtained show synergistic inhibitory activity for the mixtures:

i) 0.0625% of A and 0.0158% of B1, i.e. ratio A/B1 = 3.95
ii) 0.125% of A and 0.0158% of B1, i.e. ratio A/B1 = 7.91
iii) 0.25% of A and 0.0158% of B1, i.e. ratio A/B1 = 15.82
iv) 0.5% of A and 0.0158% of B1, i.e. ratio A/B1 = 31.64
v) 0.5% of A and 0.0079% of B1, i.e. ratio A/B1 = 63.29

## Claims

1. Antimicrobial mixture comprising 4-(3-ethoxy-4-hydroxyphenyl)butan-2-one and an ammonium compound :
i) an alkyltrimethylammonium salt of formula $R\text{-}N^+(CH_3)_3\ X^-$ (I) in which R denotes an alkyl group containing from 20 to 24 carbon atoms, $X^-$ denotes a halide ion, in particular chloride or bromide, or a methosulfate ion.

2. Mixture according to the preceding claim, **characterized in that**, for the trimethyl ammonium salt of formula (I), R is a behenyl group.

3. Antimicrobial mixture according to either of Claim1 and 2, **characterized in that** the alkyltrimethylammonium salt (I) is behenyltrimethylammonium chloride.

4. Mixture according to one of Claims 1 to 3, **characterized in that** it comprises 4-(3-ethoxy-4-hydroxyphenyl)butan-2-one and the alkyltrimethylammonium salt of formula (I) in amounts such that the 4-(3-ethoxy-4-hydroxyphenyl)butan-2-one/salt (I) weight ratio ranges from 0.08 to 80, preferably from 0.45 to 70, and preferentially from 1.5 to 50.

5. Composition comprising, in a physiologically acceptable medium, an antimicrobial mixture according to one of Claims 1 to 4.

6. Composition according to the preceding claim, **characterized in that** it comprises at least one additional ingredient chosen from water, oils, polyols containing from 2 to 10 carbon atoms, gelling agents, surfactants, film-forming polymers, dyestuffs, fragrances, fillers, UV-screening agents, plant extracts, cosmetic and dermatological active agents, and salts.

7. Composition according to either of Claims 5 and 6, **characterized in that** the 4-(3-ethoxy-4-hydroxyphenyl)butan-2-one is present in a content ranging from 0.01% to 5% by weight relative to the total weight of the composition, preferably ranging from 0.01% to 3% by weight, and preferentially ranging from 0.01% to 2.5% by weight.

8. Non-therapeutic cosmetic treatment process for caring for and/or making up and/or cleansing keratin materials, comprising the application to said keratin materials of a composition according to any one of Claims 5 to 7.

9. Process for conserving a composition comprising a physiologically acceptable medium, in particular a cosmetic or dermatological composition, **characterized in that** it consists in incorporating into said composition an antimicrobial mixture as defined in one of Claims 1 to 4.

10. Use of an antimicrobial mixture according to any one of Claims 1 to 4, for the conservation of a composition comprising a physiologically acceptable medium.

**Patentansprüche**

1. Antimikrobielle Mischung, umfassend 4-(3-Ethoxy-4-hydroxyphenyl)butan-2-on und eine Ammoniumverbindung:
i) ein Alkyltrimethylammoniumsalz der Formel $R\text{-}N^{+}(CH_3)_3\ X^{-}$ (I), wobei R für eine Alkylgruppe mit 20 bis 24 Kohlenstoffatomen steht und $X^{-}$ für ein Halogenidion, insbesondere Chlorid oder Bromid, oder ein Methosulfat-Ion, steht.

2. Mischung nach dem vorhergehenden Anspruch, **dadurch gekennzeichnet, dass** für das Trimethylammoniumsalz der Formel (I) R für eine Behenylgruppe steht.

3. Antimikrobielle Mischung nach Anspruch 1 oder 2, **dadurch gekennzeichnet, dass** es sich bei dem Alkyltrimethylammoniumsalz (I) um Behenyltrimethylammoniumchlorid handelt.

4. Mischung nach einem der Ansprüche 1 bis 3, **dadurch gekennzeichnet, dass** sie 4-(3-Ethoxy-4-hydroxyphenyl)butan-2-on und das Alkyltrimethylammoniumsalz der Formel (I) in solchen Mengen umfasst, dass das Gewichtsverhältnis von 4-(3-Ethoxy-4-hydroxyphenyl)butan-2-on zu Salz (I) im Bereich von 0,08 bis 80, vorzugsweise von 0,45 bis 70 und bevorzugt von 1,5 bis 50 liegt.

5. Zusammensetzung, die in einem physiologisch unbedenklichen Medium eine antimikrobielle Mischung nach einem der Ansprüche 1 bis 4 umfasst.

6. Zusammensetzung nach dem vorhergehenden Anspruch, **dadurch gekennzeichnet, dass** sie mindestens einen zusätzlichen Inhaltsstoff umfasst, der aus Wasser, Ölen, Polyolen mit 2 bis 10 Kohlenstoffatomen, Geliermitteln, Tensiden, filmbildenden Polymeren, Farbstoffen, Duftstoffen, Füllstoffen, UV-Filtersubstanzen, Pflanzenextrakten, kosmetischen und dermatologischen Wirkstoffen und Salzen ausgewählt ist.

7. Zusammensetzung nach einem der Ansprüche 5 und 6, **dadurch gekennzeichnet, dass** das 4-(3-Ethoxy-4-hydroxyphenyl)butan-2-on in einem Gehalt im Bereich von 0,01 bis 5 Gew.-%, bezogen auf das Gesamtgewicht der Zusammensetzung, vorzugsweise im Bereich von 0,01 bis 3 Gew.-% und bevorzugt im Bereich von 0,01 bis 2,5 Gew.-% vorliegt.

8. Nichttherapeutisches kosmetisches Behandlungsverfahren zum Pflegen und/oder Schminken und/oder Reinigen

von Keratinmaterialien, umfassend das Aufbringen einer Zusammensetzung nach einem der Ansprüche 5 bis 7 auf die Keratinmaterialien.

**9.** Verfahren zum Konservieren einer Zusammensetzung, die ein physiologisch unbedenkliches Medium umfasst, insbesondere einer kosmetischen oder dermatologischen Zusammensetzung, **dadurch gekennzeichnet, dass** es aus dem Einarbeiten einer antimikrobiellen Mischung gemäß einem der Ansprüche 1 bis 4 in die Zusammensetzung besteht.

**10.** Verwendung einer antimikrobiellen Mischung gemäß einem der Ansprüche 1 bis 4 zur Konservierung einer Zusammensetzung, die ein physiologisch unbedenkliches Medium umfasst.

**Revendications**

**1.** Mélange antimicrobien comprenant de la 4-(3-éthoxy-4-hydroxyphényl)butan-2-one et composé ammonium choisi parmi :
i) un sel d'alkyltriméthylammonium de formule $R\text{-}N^+(CH_3)_3X^-$ (I) dans laquelle R désigne un groupe alkyle contenant de 20 à 24 atomes de carbone, X- désigne un ion halogénure, en particulier chlorure ou bromure, ou un ion méthosulfate.

**2.** Mélange selon la revendication précédente, **caractérisé en ce que**, pour le sel de triméthylammonium de formule (I), R est un groupe béhényle.

**3.** Mélange antimicrobien selon l'une ou l'autre parmi les revendications 1 ou 2, **caractérisé en ce que** le sel d'alkyltriméthylammonium (I) est le chlorure de béhényltriméthylammonium.

**4.** Mélange selon l'une des revendications 1 à 3, **caractérisé en ce qu'**il comprend de la 4-(3-éthoxy-4-hydroxyphényl)butan-2-one et le sel d'alkyltriméthylammonium de formule (I) en quantités telles que le rapport pondéral 4-(3-éthoxy-4-hydroxyphényl)butan-2-one/sel (I) se situe dans la plage de 0,08 à 80, de préférence de 0,45 à 70, et préférentiellement de 1,5 à 50.

**5.** Composition comprenant, dans un milieu physiologiquement acceptable, un mélange antimicrobien selon l'une des revendications 1 à 4.

**6.** Composition selon la revendication précédente, **caractérisée en ce qu'**elle comprend au moins un ingrédient supplémentaire choisi parmi l'eau, les huiles, les polyols contenant de 2 à 10 atomes de carbone, les agents gélifiants, les tensioactifs, les polymères filmogènes, les matières colorantes, les fragrances, les charges, les agents écrans UV, les extraits végétaux, les agents actifs cosmétiques et dermatologiques, et les sels.

**7.** Composition selon l'une ou l'autre des revendications 5 ou 6, **caractérisée en ce que** la 4-(3-éthoxy-4-hydroxyphényl)butan-2-one est présente en une teneur dans la plage de 0,01 % à 5 % en poids, par rapport au poids total de la composition, de préférence dans la plage de 0,01 % à 3 % en poids, et préférentiellement dans la plage de 0,01 à 2,5 % en poids.

**8.** Procédé de traitement cosmétique non thérapeutique de soin et/ou de maquillage et/ou de nettoyage de matières kératiniques comprenant l'application sur lesdites matières kératiniques d'une composition selon l'une quelconque des revendications 5 à 7.

**9.** Procédé de conservation d'une composition comprenant un milieu physiologiquement acceptable, en particulier d'une composition cosmétique ou dermatologique, **caractérisé en ce qu'**il consiste à incorporer dans ladite composition un mélange antimicrobien tel que défini dans l'une des revendications 1 à 4.

**10.** Utilisation d'un mélange antimicrobien selon l'une quelconque des revendications 1 à 4, pour la conservation d'une composition comprenant un milieu physiologiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011039445 A
- US 2005019431 A1
- US 2015265666 A1


### Non-patent literature cited in the description


- **P GILBERT et al.** Antimicrobial activity of some alkyltrimethylammonium bromides. *Letters in Applied Micriobiology,* 1985, vol. 1, 101-104
- **F.C. KULL ; P.C. EISMAN ; H.D. SYLWESTROWKA ; R.L. MAYER.** *Applied Microbiology,* 1961, vol. 9, 538-541